(19) Europäisches Patentamt / European Patent Office / Office européen des brevets

(11) **EP 2 303 008 B1**

(12) **EUROPÄISCHE PATENTSCHRIFT**

(45) Veröffentlichungstag und Bekanntmachung des Hinweises auf die Patenterteilung:
**06.01.2016 Patentblatt 2016/01**

(21) Anmeldenummer: **09779955.5**

(22) Anmeldetag: **25.06.2009**

(51) Int Cl.:
*A01N 25/10* *(2006.01)*          *A01N 59/00* *(2006.01)*
*A01N 59/16* *(2006.01)*          *A01N 59/20* *(2006.01)*
*A61L 27/00* *(2006.01)*          *A01P 1/00* *(2006.01)*

(86) Internationale Anmeldenummer:
**PCT/EP2009/057992**

(87) Internationale Veröffentlichungsnummer:
**WO 2010/006915 (21.01.2010 Gazette 2010/03)**

(54) **VERFAHREN ZUR HERSTELLUNG EINES KOMPOSITWERKSTOFFS MIT ANTIMIKROBIELLER WIRKUNG**

PROCESS FOR PRODUCTION OF A COMPOSITE MATERIAL HAVING ANTIMICROBIAL ACTIVITY

PROCÉDÉ DE PRODUCTION D'UN MATÉRIAU COMPOSITE À ACTION ANTIMICROBIENNE

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **15.07.2008 DE 102008033224**

(43) Veröffentlichungstag der Anmeldung:
**06.04.2011 Patentblatt 2011/14**

(73) Patentinhaber: **Bio-Gate AG**
**90411 Nürnberg (DE)**

(72) Erfinder: **STEINRÜCKE, Peter**
**90461 Nürnberg (DE)**

(74) Vertreter: **Gassner, Wolfgang**
**Dr. Gassner & Partner mbB**
**Patentanwälte**
**Marie-Curie-Strasse 1**
**91052 Erlangen (DE)**

(56) Entgegenhaltungen:
**WO-A1-02/17984     WO-A1-03/024494**

• **'Heraeus. Palacos®R', MANUFACTURER. HERAUS MEDICAL GMBH., GERMANY Seiten 1 - 112**

EP 2 303 008 B1

**Beschreibung**

Die Erfindung betrifft ein Verfahren zur Herstellung eines Kompositwerkstoffs mit antimikrobieller Wirkung.

Die US 5,837,275 offenbart eine antimikrobielle Beschichtung, bei der aus Silber hergestellte Nanopartikel mittels Sputtern auf eine zu beschichtende Oberfläche aufgebracht werden. Aus Nanopartikeln hergestellte Pulver haben die nachteilige Eigenschaft, dass sie in einer Flüssigkeit oder einem Harz nur äußerst schwer homogen dispergiert werden können. Abgesehen davon neigen Nanopartikel dazu, relativ harte Agglomerate zu bilden. Auch das wirkt einer homogenen Verteilung der Nanopartikel in einem Kompositwerkstoff entgegen.

Die WO 02/17984 A1 beschreibt ein antimikrobielles Material zum Implantieren in Knochen. Zur Herstellung des Materials werden poröse Silberaggregate zunächst in ein Kunststoffharz eingerührt und vollständig mit dem Kunststoffharz infiltriert. Anschließend wird das Kunststoffharz ausgehärtet. Bei der Herstellung des bekannten Kompositwerkstoffs tritt das Problem auf, dass die Silberaggregate der Schwerkraft folgend stets die Neigung haben, sich am Boden eines das Kunststoffharz aufnehmenden Behälters anzureichern. Dem kann zwar durch Erhöhen der Viskosität des Kunststoffharzes entgegengewirkt werden. In diesem Fall tritt allerdings das Problem auf, dass die Silberaggregate nicht vollständig infiltriert werden. Das führt wiederum zu einer verminderten antimikrobiellen Wirksamkeit des Kompositwerkstoffs.

Aufgabe der Erfindung ist es, die Probleme nach dem Stand der Technik zu beseitigen. Es soll insbesondere ein Verfahren zur Herstellung eines Kompositwerkstoffs mit antimikrobieller Wirkung angegeben werden, welches einfach und kostengünstig durchführbar ist. Nach einem weiteren Ziel der Erfindung soll ein möglichst einfach herstellbarer Kompositwerkstoff mit verbesserter antimikrobieller Wirksamkeit angegeben werden. Diese Aufgabe wird durch die Merkmale der Ansprüche 1, 2 und 18 gelöst. Zweckmäßige Ausgestaltungen der Erfindung ergeben sich aus den Merkmalen der Ansprüche 3 bis 17 und 19 bis 25. Nach einem ersten Aspekt der Erfindung ist ein Verfahren zur Herstellung eines Kompositwerkstoffs mit antimikrobieller Wirkung mit folgenden Schritten vorgesehen:

- Bereitstellen eines aus einem antimikrobiell wirkenden Metall hergestellten Metallpulvers, wobei das Metallpulver aus diskreten Agglomeraten mit einer Porosität von 30 bis 98% gebildet ist, wobei die Agglomerate aus über Sinterhälse fest miteinander verbundenen Primärpartikeln gebildet sind und eine offenporige schwammartige Gerüststruktur aufweisen;

- Aufschmelzen eines thermoplastischen Kunststoffs und Einstellen einer vorgegebenen Viskosität;

- Mischen des Metallpulvers mit dem aufgeschmolzenen thermoplastischen Kunststoff in einem vorgegebenen Mengenverhältnis; und

- Abkühlen der Mischung, wobei das Metallpulver fest mit einer durch den Kunststoff gebildeten Matrix verbunden wird.

Die erfindungsgemäßen Agglomerate weisen eine feste schwammartige Gerüststruktur auf. Die schwammartige Gerüststruktur umgibt ein offenes Porenvolumen. Eine offene Porosität im Sinne der vorliegenden Erfindung ist definiert durch

$$\Theta = (1 - \rho/\rho_0) * 100\%,$$

wobei

$\rho$ die Rohdichte des Metalls und

$\rho_0$ die Reindichte des Metalls ist.

Die erfindungsgemäßen Agglomerate haben den Vorteil, dass deren Gerüststruktur bei einer Einarbeitung in eine thermoplastische Schmelze nicht zerstört wird. D. h. die Porosität der Agglomerate bleibt erhalten. Von den erfindungsgemäßen Agglomeraten sind abzugrenzen Aggregate, welche sich zufällig aus Nanoteilchen bilden und im Wesentlichen nicht durch feste Materialbrücken, sondern durch elektrostatische Anziehungskräfte miteinander verbunden sind. Derartige Aggregate ändern ihre Struktur bei der Einarbeitung in eine thermoplastische Schmelze. Insbesondere weisen sie im eingearbeiteten Zustand nicht die mit den erfindungsgemäßen Agglomeraten erzielbare Porosität auf.

Unter Verwendung der erfindungsgemäßen Agglomerate kann auf überraschend einfache und kostengünstige Weise ein Kompositwerkstoff mit hoher antimikrobieller Wirksamkeit hergestellt werden.

Erfindungsgemäß wird ein Metallpulver verwendet, dessen Partikel aus diskreten porösen Agglomeraten gebildet sind. Damit eignet sich der vorgeschlagene Kompositwerkstoff insbesondere auch zur Herstellung von Implantaten, Kathetern und dergleichen. Die vorgeschlagenen Agglomerate haben keine unerwünschte zytotoxische Wirkung. Gleichzeitig weisen sie eine hohe innere Oberfläche auf, welche eine Freisetzung einer relativ hohen Rate an eine antimikrobielle Wirkung hervorrufenden Metallionen ermöglicht. Indem erfindungsgemäß zur Herstellung des Kompositwerkstoffs ein thermoplastischer Kunststoff verwendet wird, kann eine besonders gleichmäßige und homogene Verteilung des Metallpulvers im Kompositwerkstoff erreicht werden.

Mit dem vorgeschlagenen Verfahren kann zunächst ein Halbzeug hergestellt werden. Dabei kann es sich um ein Granulat, um Stangen, Platten oder dergleichen handeln. Das Halbzeug kann in einem weiteren Verfahrensschritt zu einem gewünschten Formkörper weiterverarbeitet werden.

Nach einem weiteren Aspekt der Erfindung ist ein Verfahren zur Herstellung eines antimikrobielle Eigenschaften aufweisenden Kompositwerkstoffs mit folgenden Schritten vorgesehen:

- Bereitstellen eines aus einem antimikrobiell wirkenden Metall hergestellten Metallpulvers, wobei das Metallpulver aus diskreten Agglomeraten mit einer Porosität von 30 bis 98% gebildet ist, wobei die Agglomerate aus über Sinterhälse fest miteinander verbundenen Primärpartikeln gebildet sind und offenporige schwammartige Struktur aufweisen;

- Bereitstellen eines aus einem thermoplastischen Kunststoff hergestellten Kunststoffpulvers;

- Mischen des Metallpulvers und des Kunststoffpulvers in einem vorgegebenen Mengenverhältnis;

- Aufheizen einer aus dem Metallpulver und dem Kunststoffpulver gebildeten Mischung auf eine Temperatur im Bereich der Schmelztemperatur des Kunststoffpulvers; und

- Abkühlen der Mischung, wobei das Metallpulver fest mit einer durch den thermoplastischen Kunststoff gebildeten Matrix verbunden wird.

Im Gegensatz zum oben vorgeschlagenen Verfahren nach dem ersten Aspekt der Erfindung wird nach dem zweiten Aspekt der Erfindung zunächst eine Mischung aus dem Metallpulver und dem Kunststoffpulver hergestellt. Eine solche Mischung lässt sich einfach herstellen. Sie kann als Zwischenprodukt zwischengelagert werden. Daraus können Halbzeuge oder Formteile hergestellt werden. Zu diesem Zweck wird die Mischung aus dem Metallpulver und dem Kunststoffpulver auf eine Temperatur im Bereich der Schmelztemperatur des Kunststoffpulvers aufgeheizt.

Nach einer Ausgestaltung des Verfahrens kann vor dem Schritt des Aufheizens der Mischung aus der Mischung mittels Pressen ein Presskörper hergestellt werden. Der Presskörper kann ein Formkörper sein, der anschließend durch die erfindungsgemäße Wärme- und Druckbehandlung verdichtet wird.

Als zweckmäßig hat es sich erwiesen, dass eine mittlere Korngröße der das Kunststoffpulver bildenden Kunststoffpartikel etwa einer mittleren Korngröße der Agglomerate entspricht. Das ermöglicht die Herstellung einer besonders homogenen Mischung.

Die nachfolgend beschriebenen Ausgestaltungen können auf beide Aspekte des erfindungsgemäßen Verfahrens angewendet werden.

Nach einer vorteilhaften Ausgestaltung wird auf die Mischung ein vom Umgebungsdruck verschiedener Druck ausgeübt. Bei dem Druck kann es sich um einen Druck handeln, der größer als der Umgebungsdruck ist. Damit wird die Schmelze des thermoplastischen Kunststoffs bzw. die thermoplastische Schmelze in den offenen Porenraum der Agglomerate gedrückt. Bei dem Druck kann es sich aber auch um einen Unterdruck handeln, d. h. einen Druck, der kleiner ist als der Umgebungsdruck. Unter dem Einfluss eines Unterdrucks entweicht aus der Mischung, insbesondere aus dem Porenraum der Agglomerate, die darin befindliche Luft. Auch das unterstützt die Infiltration der thermoplastischen Schmelze in den Porenraum der Agglomerate. Sofern ein Über- oder Unterdruck auf die Mischung ausgeübt wird, ist darauf zu achten, dass dieser so gewählt wird, dass die schwammartige Gerüststruktur der Agglomerate nicht zerstört wird. Die Größe des aufzubringenden Drucks hängt von der Struktur der Agglomerate, der Viskosität der thermoplastischen Schmelze, der Art und der Menge von Additiven und dergleichen ab.

Nach einer Ausgestaltung ist vorgesehen, dass der Schritt des Aufheizens und des Ausübens eines Drucks gleichzeitig durchgeführt werden. D. h. die Mischung wird zweckmäßigerweise heiß gepresst. Damit kann eine besonders effektive Verdichtung des Materials erreicht werden.

Nach einem besonders vorteilhaften Ausgestaltungsmerkmal kann der Druck auf die Mischung auch bei einer Formgebung mittels Spritzguss oder Extrusion aufgebracht werden. Zu diesem Zweck kann die Mischung beispielsweise zunächst in einem Compounder mit axial bewegbarer Schnecke hergestellt werden. Nach dem Herstellen der Mischung kann sodann durch eine Axialbewegung der Schnecke ein Druck auf die Mischung ausgeübt und damit die Mischung durch ein Mundstück extrudiert werden. Durch eine Axialbewegung der Schnecke ist es auch möglich, die Mischung unter Druck in eine Spritzgussform zu schießen.

Nach einer weiteren Ausgestaltung ist es auch möglich, während des Aufheizens und/oder der Ausübung eines Drucks die Mischung zu evakuieren. Damit gelingt die Herstellung eines besonders dichten und nahezu porenfreien Kompositwerkstoffs.

Nach einer weiteren Ausgestaltung ist der thermoplastische Kunststoff aus der folgenden Gruppe ausgewählt: Acrylonitril-Butadien-Styren (ABS), Acryl, Zelluloid, Zellulose-Azetat, Ethylen-Vinyl-Acetat (EVA), Ethylen-Vinyl-Alkohol (EVAL), Fluoroplaste (PTFE, FEP, PFA, CTFE, ECTFE, ETFE), Ionomere, Kydex®, Flüssigkristallpolymer (LCP), Polyacetal (POM oder Acetal), Polyacrylate (Acrylic), Polyacrylonitril (PAN oder Acrylonitril), Polyamid (PA), Polyamid-Imid (PAI), Polyacryletherketon (PAEK), Polybutadien (PBD), Polybutylen (PB), Polybutylenterephthalat (PBT), Polycaprolacton (PCL), Polychlorotrifluoroethylen (PCTFE), Polyethylenterephthalat (PET), Polycyclohexylendimethylenterephthalat (PCT), Polykarbonat (PC), Polyhydroxyalkanoat (PHAs), Polyketon (PK), Polyester, Polyethylen (PE), Polyetheretherketon (PEEK), Polyetherimid (PEI), Polyethersulfon (PES), Polyethylenchlorinat (PEC), Polyimid (PI), Polylactid (PLA), Polymethylpenten (PMP), Polyphenylenoxid (PPO), Polyphenylensulfid (PPS), Polyphthalamid (PPA), Polypropylen (PP), Polystyren (PS), Polysulfon (PSU), Polyvinylchlorid (PVC), Polyvinylidenchlorid (PVDC), Spectralon®. Der insbesondere unter Verwendung der vorgenannten thermoplastischen Kunststoffe hergestellte Kompositwerkstoff ist wegen seiner antimikrobiellen Wirkung vielseitig verwendbar. Er eignet sich insbesondere als Material zur Herstellung von Kühlschränken, Drug-Delivery-Systemen, mechanischen Schockabsorbern in Schuhen, Isoliermaterial, Gefäßimplantaten, funktionellen Textilien, technischen Textilien, Schläuchen, Kabeln, Laminaten und Fenstern, Membranen, Dichtungen, Instrumentenkonsolen, Türverkleidungen, Sitzbezügen, Jalousien, Tabletts, Sicherheitshelmen, Innenverkleidungen von Flugzeugen, Entlüftungssystemen, Implantaten, intraokulare Linsen, künstlichen Zähnen, Zahnfüllungen, Klebstoffen, künstlichen Fingernägeln, Superabsorber, Harnblasenkatheter, Nahtmaterial, Textilfasern, Katheterschläuchen, Komponenten für Dialysegeräte, Spritzen, Herzklappen, Teppichbodenfasern, Angelschnüren, Strumpfhosen, Borsten für Zahnbürsten, resorbierbares Nahtmaterial, künstlichen Blutgefäßen, Sehnen- und Bänderersatz, Verpackungsmaterial, chirurgische Befestigungsmittel wie Schrauben, Knochenplatten, Knochenplattensysteme, chirurgische Netze, kardiovaskulare Flicken, Stents, Gewebereparaturvorrichtungen, Meniskusaufbauvorrichtungen, Hautersatzstoffe, Knochenersatzstoffe, Wundauflagen, Nervenersatzstoff, Gelenkpfannen für Hüftgelenkprothesen, künstliche Knieprothesen, Hüftgelenke, zur Herstellung von Ultraschallköpfen, Komponenten für Blutoxygeneratoren und Nierendialyse, Fingergelenk-Prothesen, extrakorporale Blutschläuche, Blutbeutel, Beutel für intravenöse Anwendungen, und dergleichen.

Im Hinblick auf eine besonders effiziente antimikrobielle Wirkung hat sich die Verwendung von Agglomeraten als zweckmäßig erwiesen, deren mittlere Korngröße im Bereich von 1 bis 30 µm, vorzugsweise im Bereich von 5 bis 25 µm, ist. Agglomerate mit der vorgeschlagenen mittleren Korngröße lassen sich gut in einer thermoplastischen Schmelze dispergieren. Es kann damit ein homogener Kompositwerkstoff hergestellt werden.

Die Agglomerate weisen zweckmäßigerweise eine Dichte im Bereich von 0,4 bis 1,8 g/cm³ auf. Die Dichte der Agglomerate ist ähnlich der Dichte des thermoplastischen Kunststoffs. Damit kann vorteilhafterweise eine durch Schwerkraft bedingte Entmischung des Metallpulvers vermieden werden. Das Metallpulver verteilt sich gleichmäßig in der Mischung und infolgedessen auch im daraus hergestellten Kompositwerkstoff.

Die verwendeten Agglomerate weisen zweckmäßigerweise eine Porosität von 70 bis 98% oder von 80 bis 95% auf. Zu deren Herstellung ist also nur eine relativ kleine Menge an antimikrobiell wirkendem Metall erforderlich.

Die Primärpartikel können eine mittlere Korngröße im Bereich von 10 bis 100 nm aufweisen. Das Metallpulver bzw. derartige Agglomerate können mittels Inertgasverdampfung hergestellt werden. Das antimikrobiell wirkende Metall enthält zweckmäßigerweise eines oder mehrere der folgenden Elemente als Hauptbestandteil: Ag, Au, Pt, Pd, Ir, Sn, Cu, Sb, Zn. Vorzugsweise enthält das antimikrobiell wirkende Metall im Wesentlichen Ag.

Nach einer weiteren besonders vorteilhaften Ausgestaltung können die Agglomerate vor dem Schritt des Herstellens einer Mischung mit dem thermoplastischen Kunststoff mit einer Flüssigkeit, einem Wachs oder einem Polymer infiltriert werden. Derartig infiltrierte Agglomerate sind besonders druckfest. D. h. sie können bei einem hohen Druck in eine thermoplastische Schmelze eingearbeitet und insbesondere auch mittels Extrusion oder im Spritzgussverfahren weiter verarbeitet werden. Der vorgeschlagene Verfahrensschritt des Infiltrierens wird insbesondere dann zum Einsatz kommen, wenn die Agglomerate in eine thermoplastische Schmelze mit einer hohen Viskosität eingearbeitet oder wenn aus verfahrenstechnischen Gründen die Mischung einem hohen Druck ausgesetzt werden soll. Die zur Infiltration der Agglomerate verwendete Flüssigkeit, das Wachs oder das Polymer sind so gewählt, dass damit die Materialeigenschaften des thermoplastischen Kunststoffs nicht negativ beeinflusst werden. Insbesondere kann es sich bei der infiltrierten Flüssigkeit oder dem infiltrierten Wachs um Stoffe handeln, welche üblicherweise als Additive, beispielsweise zur Verflüssigung thermoplastischer Schmelzen, verwendet werden. Bei dem Polymer handelt es sich zweckmäßigerweise um einen Stoff, der mit dem jeweils verwendeten thermoplastischen Kunststoff sich verbindet oder darin gelöst wird. Das zur Infiltration verwendete fließfähige Material kann auch gefärbt sein. Damit ist es möglich, das Erscheinungsbild eines die Agglomerate enthaltenden Kompositwerkstoffs zu verändern.

In verfahrenstechnischer Hinsicht hat es sich weiter als besonders zweckmäßig erwiesen, zur Herstellung der Mischung einen, vorzugsweise beheizbaren, Compounder zu verwenden. Der Compounder kann eine axial bewegbare Schnecke aufweisen. Es auch ein Doppelschneckencompounder eingesetzt werden.

Auf die Mischung wird zweckmäßigerweise ein Druck von mehr als $0,5 * 10^5$ Pa, vorzugsweise mehr als $5 * 10^5$ Pa, ausgeübt. Die vorgenannte Druckangabe versteht sich als "Überdruck", d. h. es handelt sich um einen Druck, der zusätzlich zum herrschenden Luftdruck auf die Mischung aufgebracht wird. Der Druck kann mechanisch oder auch mit einem unter Druck stehenden Gas aufgebracht werden. Zweckmäßigerweise wird der Druck zumindest für eine Dauer von 0,1 bis 120 Sekunden auf die Mischung ausgeübt. Die angegebene Mindesthaltezeit ist erforderlich, um ein im Wesentlichen vollständiges Infiltrieren der Agglomerate mit dem thermoplastischen Kunststoff zu gewährleisten. Die Haltezeit richtet sich im Wesentlichen nach der Viskosität der Schmelze des thermoplastischen Kunststoffs. Längere Haltezeiten sind möglich.

Nach einem weiteren Aspekt der Erfindung ist ein Kompositwerkstoff mit antimikrobieller Wirkung vorgesehen, bei dem in einer aus einem thermoplastischen Kunststoff gebildeten Matrix aus einem antimikrobiell wirkenden Metall hergestellte diskrete Agglomerate mit einer Porosität von 30 bis 98% aufgenommen sind, wobei die Agglomerate aus über Sinterhälse fest miteinander verbundenen Primärpartikeln gebildet sind und eine offenporige schwammartige Gerüststruktur aufweisen.

Die Primärpartikel können eine mittlere Korngröße im Bereich von 10 bis 100 nm aufweisen. Unter dem Begriff "Sinterhals" wird eine Materialbrücke zwischen zwei benachbarten Primärpartikeln verstanden. Sinterhälse entstehen in der Frühphase des Sinterns durch Diffusionsprozesse. Derartige "Sinterhälse" sind zwar im Zusammenhang mit dem Verfahren des "Sinterns" beschrieben. Es ist aber auch möglich, dass Sinterhälse sich bei anderen Verfahren bilden, bei denen ähnliche Bedingungen wie beim Sintern herrschen.

Agglomerate mit einer schwammartigen Gerüststruktur können aber auch auf andere Weise hergestellt werden. Beispielsweise ist es möglich, Metallschmelzen unter Verwendung von Treibmitteln in geeigneter Weise aufzuschäumen. Ferner ist es möglich, eine inhomogene Mischung eines edlen und eines unedlen Metalls herzustellen und dann selektiv das unedle Metall durch Säurebehandlung aufzulösen, so dass eine aus dem edleren Metall gebildete schwammartige hochporöse Gerüststruktur zurückbleibt.

Die Agglomerate sind zweckmäßigerweise in einer Menge von 0,1 bis 5,0 Gew.% enthalten. Bereits die angegebenen geringen Mengen reichen aus, um dem Kompositmaterial eine antimikrobielle Wirkung zu verleihen.

Wegen der weiteren Ausgestaltungsmerkmale des Kompositwerkstoffs wird auf die vorangegangenen Ausführungen verwiesen. Die dort beschriebenen Merkmale können sinngemäß auch Merkmale des Kompositwerkstoffs bilden.

Mit dem erfindungsgemäßen Verfahren ist es erstmals auf relativ einfache und kostengünstige Weise möglich, thermoplastische Kompositwerkstoffe mit einem relativ hohen Schmelzpunkt mit einer antimikrobiellen Wirkung zu versehen. Herkömmliche antimikrobiell wirkende organische Zusatzstoffe haben bisher mangels ausreichender Temperaturstabilität nicht zur Herstellung von Kompositwerkstoffen mit einem hohen Schmelzpunkt verwendet werden können. Unter Verwendung der erfindungsgemäßen Agglomerate dagegen ist es möglich, selbst hochschmelzenden thermoplastischen Kunststoffen eine antimikrobielle Wirkung zu verleihen.

Nachfolgend werden Ausführungsbeispiele der Erfindung näher erläutert.

Ausführungsbeispiel 1:

Polyoxyethylen (Hostaform C 9021 GV1/10) wurde bei einer Temperatur von 190°C in einem PolyDriveThermo Haake Kneter (Firma Haake, Karlsruhe) geschmolzen. Dann wurde die Schmelze bei einer Drehzahl von 70 Umdrehungen/Minute mit 0,5 Gew.% Metallpulver versetzt. Das Metallpulver bestand aus Silberagglomeraten mit einer Porosität von etwa 80% und einer mittleren Korngröße von etwa 25 $\mu$m. Die mittlere Korngröße der Primärpartikel betrug 20 bis 50 nm.

Die Mischung wurde bei 190°C für etwa 8 Minuten gerührt. Sodann wurde die Schmelze zwischen zwei Messingplatten zu flachen Scheiben verformt und nach dem Abkühlen in einer Schneidmühle (Typ C13.20vs, der Firma Wanner Technik GmbH) zu einem Granulat mit einem mittleren Durchmesser von etwa 3 mm verarbeitet.

Zum Prüfen der antimikrobiellen Wirksamkeit des Granulats wurden 66,7 g Granulat in einem Liter einer verdünnten Natriumnitratlösung (7 mM) suspendiert und für eine Dauer von 72 Stunden bei Raumtemperatur inkubiert. Anschließend wurde mittels Voltametrie die Konzentration der Silberionen im Überstand bestimmt. Es ist festgestellt worden, dass der Überstand einen Silbergehalt von 2,1 $\mu$M pro Liter aufweist. Die gemessene Konzentration an Silberionen ist antimikrobiell wirksam.

Ausführungsbeispiel 2:

Polyurethan (Elastolan C85A10 der Firma BASF AG) wurde bei einer Temperatur von 185°C in einem PolyDriveThermo Haake Kneter (Firma Haake, Karlsruhe) geschmolzen. Anschließend wurde die Schmelze mit 0,5 Gew.% mit dem im Ausführungsbeispiel 1 beschriebenen Metallpulver versetzt. Die mit dem Metallpulver versetzte Schmelze wurde bei 70 Umdrehungen/Minute für 8 Minuten gerührt. Anschließend wurde die Schmelze zwischen zwei Messingplatten zu flachen Scheiben verformt. Die flachen Scheiben wurden nach dem Abkühlen in einer Schneidmühle (Typ C13.20vs, Firma Wanner Technik GmbH) zu einem Granulat mit einem mittleren Durchmesser von etwa 3 mm verarbeitet.

Eine wie oben beschriebene Messung der Konzentration der abgegebenen Silberionen ergab eine Konzentration von 1,6 $\mu$M Silberionen pro Liter. Eine derartige Konzentration an Silberionen ist antimikrobiell wirksam.

Ausführungsbeispiel 3:

Polyacetal (POM Delrin 500 NC010, der Firma Dupont) wurde in einem Extruder bei einer Temperatur von 214°C geschmolzen und mit 3 Gew.% des oben beschriebenen Silberpulvers versetzt. Die Schmelze wurde mit einem Durchsatz von 20 kg/Stunde bei einer Drehzahl von 370 Umdrehung/Minute und einem Betriebsdruck von 24 bar extrudiert. Aus dem extrudierten Material wurde ein Granulat hergestellt.

Eine Messung der Silberionen ergab wiederum, dass das Material antimikrobiell wirksam ist.

**Patentansprüche**

1. Kompositwerkstoff mit antimikrobieller Wirkung, bei dem in einer aus einem thermoplastischen Kunststoff gebildeten Matrix aus einem antimikrobiell wirkenden Metall hergestellte diskrete Agglomerate mit einer Porosität von 30 bis 98% aufgenommen sind, wobei die Agglomerate aus über Sinterhälse fest miteinander verbundenen Primärpartikeln gebildet sind und eine offenporige schwammartige Gerüststruktur aufweisen.

2. Kompositwerkstoff nach Anspruch 1, wobei die Agglomerate in einer Menge von 0,1 bis 5,0 Gew.% enthalten sind.

3. Kompositwerkstoff nach einem der Ansprüche 1 oder 2, wobei der thermoplastische Kunststoff aus der folgenden Gruppe ausgewählt ist: Acrylonitril-Butadien-Styren (ABS), Acryl, Zelluloid, Zellulose-Azetat, Ethylen-Vinyl-Acetat (EVA), Ethylen-Vinyl-Alkohol (EVAL), Fluoroplaste (PTFE, FEP, PFA, CTFE, ECTFE, ETFE), Ionomere, Flüssigkristallpolymer (LCP), Polyacetal (POM oder Acetal), Polyacrylate (Acrylic), Polyacrylonitril (PAN oder Acrylonitril), Polyamid (PA), Polyamid-Imid (PAI), Polyacryletherketon (PAEK), Polybutadien (PBD), Polybutylen (PB), Polybutylenterephthalat (PBT), Polycaprolacton (PCL), Polychlorotrifluoroethylen (PCTFE), Polyethylenterephthalat (PET), Polycyclohexylendimethylenterephthalat (PCT), Polykarbonat (PC), Polyhydroxyalkanoat (PHAs), Polyketon (PK), Polyester, Polyethylen (PE), Polyetheretherketon (PEEK), Polyetherimid (PEI), Polyethersulfon (PES), Polyethylenchlorinat (PEC), Polyimid (PI), Polylactid (PLA), Polymethylpenten (PMP), Polyphenylenoxid (PPO), Polyphenylensulfid (PPS), Polyphthalamid (PPA), Polypropylen (PP), Polystyren (PS), Polysulfon (PSU), Polyvinylchlorid (PVC), Polyvinylidenchlorid (PVDC).

4. Kompositwerkstoff nach einem der vorhergehenden Ansprüche, wobei eine mittlere Korngröße der Agglomerate im Bereich von 1 bis 30 $\mu$m ist.

5. Kompositwerkstoff nach einem der vorhergehenden Ansprüche, wobei die Agglomerate eine Dichte im Bereich von 0,4 bis 1,8 g/cm$^3$ aufweisen.

6. Kompositwerkstoff nach einem der vorhergehenden Ansprüche, wobei eine mittlere Korngröße der Primärpartikel im Bereich von 10 bis 100 nm ist.

7. Kompositwerkstoff nach einem der vorhergehenden Ansprüche, wobei die Agglomerate mittels Inertgasverdampfung hergestellt sind.

8. Kompositwerkstoff nach einem der vorhergehenden Ansprüche, wobei das antimikrobiell wirkende Metall eines oder mehrere der folgenden Elemente als Hauptbestandteil enthält: Ag, Au, Pt, Pd, Ir, Sn, Cu, Sb, Zn.

9. Verfahren zur Herstellung eines Kompositwerkstoffs mit antimikrobieller Wirkung, mit folgenden Schritten:

   - Bereitstellen eines aus einem antimikrobiell wirkenden Metall hergestellten Metallpulvers, wobei das Metallpulver aus diskreten Agglomeraten mit einer Porosität von 30 bis 98% gebildet ist, wobei die Agglomerate aus über Sinterhälse fest miteinander verbundenen Primärpartikeln gebildet sind und eine offenporige schwammartige Gerüststruktur aufweisen;
   - Aufschmelzen eines thermoplastischen Kunststoffs und Einstellen einer vorgegebenen Viskosität;
   - Mischen des Metallpulvers mit dem aufgeschmolzenen thermoplastischen Kunststoff in einem vorgegebenen Mengenverhältnis; und
   - Abkühlen der Mischung, wobei das Metallpulver fest mit einer durch den Kunststoff gebildeten Matrix verbunden wird.

10. Verfahren zur Herstellung eines antimikrobielle Eigenschaften aufweisenden Kompositwerkstoffs nach einem der Ansprüche 1 bis 8 mit folgenden Schritten:

   - Bereitstellen eines aus einem antimikrobiell wirkenden Metall hergestellten Metallpulvers, wobei das Metallpulver aus diskreten Agglomeraten mit einer Porosität von 30 bis 98% gebildet ist, wobei die Agglomerate aus über Sinterhälse fest miteinander verbundenen Primärpartikeln gebildet sind und eine offenporige schwammartige Gerüststruktur aufweisen;
   - Bereitstellen eines aus einem thermoplastischen Kunststoff hergestellten Kunststoffpulvers;
   - Mischen des Metallpulvers und des Kunststoffpulvers in einem vorgegebenen Mengenverhältnis;
   - Aufheizen einer aus dem Metallpulver und dem Kunststoffpulver gebildeten Mischung auf eine Temperatur im Bereich der Schmelztemperatur des Kunststoffpulvers; und
   - Abkühlen der Mischung, wobei das Metallpulver fest mit einer durch den thermoplastischen Kunststoff gebildeten Matrix verbunden wird.

**11.** Verfahren nach Anspruch 10, wobei vor dem Schritt des Aufheizens der Mischung aus der Mischung mittels Pressen ein Presskörper hergestellt wird.

**12.** Verfahren nach einem der Ansprüche 10 oder 11, wobei ein mittlerer Korndurchmesser der das Kunststoffpulver bildenden Kunststoffpartikel einem mittleren Korndurchmesser der Agglomerate entspricht.

**13.** Verfahren nach einem der Ansprüche 9 bis 12, wobei auf die Mischung ein vom Umgebungsdruck verschiedener Druck ausgeübt wird.

**14.** Verfahren nach einem der Ansprüche 9 bis 13, wobei der Schritt der Aufheizens und des Ausübens des Drucks gleichzeitig durchgeführt werden.

**15.** Verfahren nach einem der Ansprüche 9 bis 14, wobei der Druck auf die Mischung bei einer Formgebung mittels Spritzguss oder Extrusion aufgebracht wird.

**16.** Verfahren nach einem der Ansprüche 9 bis 15, wobei die Agglomerate vor dem Schritt des Herstellens einer Mischung mit dem thermoplastischen Kunststoff mit einer Flüssigkeit, einem Wachs oder einem Polymer infiltriert werden.

**17.** Verfahren nach einem der Ansprüche 9 bis 16, wobei zur Herstellung der Mischung ein beheizbarer Compounder verwendet wird.

**18.** Verfahren nach einem der Ansprüche 9 bis 17, wobei auf die Mischung ein Druck von mehr als $0{,}5 * 10^5$ Pa ausgeübt wird.

**19.** Verfahren nach einem der Ansprüche 9 bis 18, wobei der Druck für eine Dauer von zumindest 0,1 bis 120 Sekunden auf die Mischung ausgeübt wird.

**Claims**

**1.** A composite material having antimicrobial activity for which discrete agglomerates having a porosity of 30 to 98% and being made from an antimicrobial-acting metal are held in a matrix created from a thermoplastic synthetic material, wherein the agglomerates are created from primary particles which are firmly connected with each other via sinter necks, which agglomerates have an open porous spongy framework structure.

**2.** The composite material as defined in claim 1, wherein the agglomerates are contained in an amount of 0.1 to 5.0 percent by weight.

**3.** The composite material as defined in one of the claims 1 or 2, where the thermoplastic synthetic material is selected from the following group: acrylonitrile butadiene styrene (ABS), acrylic, celluloid, cellulose acetate, ethylene vinyl acetate (EVA), ethylene vinyl alcohol (EVAL), fluoroplasts (PTFE, FEP, PFA, CTFE, ECTFE, ETFE), ionomers, liquid crystal polymer (LCP), polyacetal (POM or acetal), polyacrylates (acrylic), polyacrylonitrile (PAN or acrylonitrile), polyamide (PA), polyamide imide (PAI), polyacrylic ether ketone (PAEK), polybutadiene (PBD), polybutylene (PB), polybutylene terephthalate (PBT), polycaprolactone (PCL), polychlorotrifluoroethylene (PCTFE), polyethylene terephthalate (PET), polycyclohexylendimethylen terephthalate (PCT), polycarbonate (PC), polyhydroxyalkanoate (PHAs), polyketone (PK), polyester, polyethylene (PE), polyetheretherketone (PEEK), polyetherimide (PEI), polyethersulfone (PES), polyethylenchlorinate (PEC), polyimide (PI), polylactide (PLA), polymethylpenten (PMP), polyphenylene oxide (PPO), polyphenylene sulfide (PPS), polyphthalamide (PPA), polypropylene (PP), polystyrene (PS), polysulfone (PSU), polyvinyl chloride (PVC), polyvinylidene chloride (PVDC).

**4.** The composite material as defined in one of the preceding claims, where a medium grain size of the agglomerates is in the range from 1 to 30 $\mu$m.

**5.** The composite material as defined in one of the preceding claims, wherein the agglomerates have a density in the range from 0.4 to 1.8 g/cm$^3$.

**6.** The composite material as defined in one of the preceding claims, wherein a medium grain size of the primary particles is in the range from 10 to 100 nm.

**7.** The composite material as defined in one of the preceding claims, wherein the agglomerates are made via inert gas vaporization.

**8.** The composite material as defined in one of the preceding claims, wherein the antimicrobial-acting metal contains one or more of the following elements as the main component: Ag, Au, Pt, Pd, Ir, Sn, Cu, Sb, Zn.

**9.** A process for production of a composite material having antimicrobial activity, with the following steps:

- Provision of a metal powder made from an antimicrobial-acting metal, wherein the metal powder is created from discrete agglomerates having a porosity of 30 to 98%, wherein the agglomerates are created from primary particles which are firmly connected with each other via sinter necks, which agglomerates have an open porous spongy framework structure;
- Melting a thermoplastic synthetic material and setting a specified viscosity;
- Mixing the metal powder with the melted thermoplastic synthetic material in a specified proportion; and
- Cooling off the mixture, wherein the metal powder is firmly connected with a matrix created by the synthetic material.

10. A process for production of a composite material as defined in one of claims 1 to 8, which composite material has antimicrobial properties, with the following steps:

- Provision of a metal powder made from an antimicrobial-acting metal, wherein the metal powder is created from discrete agglomerates having a porosity of 30 to 98%, wherein the agglomerates are created from primary particles which are firmly connected with each other via sinter necks, which agglomerates have an open porous spongy framework structure;
- Provision of a synthetic powder made from a thermoplastic synthetic material;
- Mixing of the metal powder and the synthetic powder in a specified proportion;
- Heating up a mixture created from the metal powder and the synthetic powder to a temperature in the range of the melting temperature of the synthetic powder; and
- Cooling off the mixture, wherein the metal powder is firmly connected with a matrix created by the thermoplastic synthetic material.

11. The process as defined in claim 10, wherein a pressed body is pressed out of the mixture before the step of heating up the mixture.

12. The process as defined in one of the claims 10 or 11, wherein a medium grain diameter of the synthetic particles which create the synthetic powder corresponds to a medium grain diameter of the agglomerates.

13. The process as defined in one of the claims 9 to 12, wherein a pressure different from the surrounding pressure is exerted on the mixture.

14. The process as defined in one of the claims 9 to 13, wherein the step of heating up and exerting the pressure are performed at the same time.

15. The process as defined in one of the claims 9 to 14, wherein the pressure is applied to the mixture during shaping via injection molding or extrusion.

16. The process as defined in one of the claims 9 to 15, wherein the agglomerates are infiltrated with a fluid, a wax or a polymer before the step of making a mixture with the thermoplastic synthetic material.

17. The process as defined in one of the claims 9 to 16, wherein a heatable compounder is used to make the mixture.

18. The process as defined in one of the claims 9 to 17, wherein a pressure of more than $0.5 * 10^5$ Pa is exerted on the mixture.

19. The process as defined in one of the claims 9 to 18, wherein the pressure is exerted on the mixture for a duration of at least 0.1 to 120 seconds.

**Revendications**

1. Matériau composite à action antimicrobienne, pour lequel des agglomérats discrets fabriqués à partir d'un métal à action antimicrobienne avec une porosité de 30 à 98 % sont reçus dans une matrice formée d'un plastique thermoplastique, dans lequel les agglomérats sont formés de particules primaires reliées solidement entre elles par le biais de ponts de frittage et présentent une structure de squelette à pores ouverts, de type éponge.

2. Matériau composite selon la revendication 1, dans lequel les agglomérats sont contenus en une quantité de 0,1 à 5,0 % en poids.

3. Matériau composite selon une des revendications 1 ou 2, dans lequel le plastique thermoplastique est sélectionné dans le groupe suivant : acrylonitrile-butadiène-styrène (ABS), acrylique, celluloïd, acétate de cellulose, éthylène-acétate de vinyle (EVA), éthylène-alcool vinylique (EVAL), fluoroplastiques (PTFE, FEP, PFA, CTFE, ECTFE, ETFE), ionomères, polymère cristal liquide (LCP), polyacétal (POM ou acétal), polyacrylates (acrylique), polyacrylonitrile (PAN ou acrylonitrile), polyamide (PA), polyamide-imide (PAI), polyacryléthercétone (PAEK), polybutadiène (PBD), polybutylène (PB), polybutylène téréphtalate (PBT), polycaprolactone (PCL), polychlorotrifluoroéthylène (PCTFE), polyéthylène téréphtalate (PET), polycyclohexylènediméthylène téréphtalate (PCT), polycarbonate (PC), polyhydroxyalcanoate (PHA), polycétone (PK), polyester, polyéthylène (PE), polyétheréthercétone (PEEK), polyétherimide (PEI), polyéthersulfone (PES), polyéthylène chlorinate (PEC), polyimide (PI), acide polylactique (PLA), polyméthylpentène (PMP), polyphénylène oxyde (PPO), polyphénylène sulfide (PPS), polyphtalamide (PPA), polypropylène (PP), polystyrène (PS), polysulfone (PSU), chlorure de polyvinyle (PVC), chlorure de polyvinylidène (PVDC).

4. Matériau composite selon une des revendications précédentes, dans lequel une granulométrie moyenne des agglomérats est dans la plage de 1 à 30 μm.

5. Matériau composite selon une des revendications précédentes, dans lequel les agglomérats présentent une densité dans la plage de 0,4 à 1,8 g/cm$^3$.

6. Matériau composite selon une des revendications précédentes, dans lequel une granulométrie moyenne des particules primaires est dans la plage de 10 à 100 nm.

7. Matériau composite selon une des revendications précédentes, dans lequel les agglomérats sont fabriqués au moyen d'une vaporisation de gaz inerte.

8. Matériau composite selon une des revendications précédentes, dans lequel le métal à action antimicrobienne contient un ou plusieurs des éléments suivants comme constituant principal : Ag, Au, Pt, Pd, Ir, Sn, Cu, Sb, Zn.

9. Procédé de fabrication d'un matériau composite à action antimicrobienne, avec les étapes suivantes :

- mise à disposition d'une poudre métallique fabriquée à partir d'un métal à action antimicrobienne, dans lequel la poudre métallique est formée d'agglomérats discrets avec une porosité de 30 à 98 %, dans lequel les agglomérats sont formés de particules primaires reliées solidement entre elles par le biais de ponts de frittage et présentent une structure de squelette à pores ouverts, de type éponge ;
- fonte d'un plastique thermoplastique et réglage d'une viscosité prédéfinie ;
- mélange de la poudre métallique avec le plastique thermoplastique fondu dans une proportion prédéfinie ; et
- refroidissement du mélange, dans lequel la poudre métallique est reliée solidement à une matrice formée par le plastique.

10. Procédé de fabrication d'un matériau composite présentant des propriétés antimicrobiennes selon une des revendications 1 à 8, avec les étapes suivantes :

- mise à disposition d'une poudre métallique fabriquée à partir d'un métal à action antimicrobienne, dans lequel la poudre métallique est formée d'agglomérats discrets avec une porosité de 30 à 98 %, dans lequel les agglomérats sont formés de particules primaires reliées solidement entre elles par le biais de ponts de frittage et présentent une structure de squelette à pores ouverts, de type éponge ;
- mise à disposition d'une poudre plastique fabriquée à partir d'un plastique thermoplastique ;
- mélange de la poudre métallique et de la poudre plastique dans une proportion prédéfinie ;
- chauffage d'un mélange formé de la poudre métallique et de la poudre plastique à une température dans la plage de la température de fusion de la poudre plastique ; et
- refroidissement du mélange, dans lequel la poudre métallique est reliée solidement à une matrice formée par le plastique thermoplastique.

11. Procédé selon la revendication 10, dans lequel un comprimé est fabriqué à partir du mélange au moyen d'un pressage avant l'étape de chauffage du mélange.

12. Procédé selon une des revendications 10 ou 11, dans lequel un diamètre moyen de grain des particules plastiques formant la poudre plastique correspond à un diamètre moyen de grain des agglomérats.

13. Procédé selon une des revendications 9 à 12, dans lequel une pression différente de la pression environnante est exercée sur le mélange.

14. Procédé selon une des revendications 9 à 13, dans lequel l'étape de chauffage et d'exercice de la pression sont effectuées en même temps.

15. Procédé selon une des revendications 9 à 14, dans lequel la pression sur le mélange est appliquée lors d'un façonnage au moyen d'un moulage par injection ou d'une extrusion.

16. Procédé selon une des revendications 9 à 15, dans lequel les agglomérats sont infiltrés avec un liquide, une cire ou un polymère avant l'étape de fabrication d'un mélange avec le plastique thermoplastique.

17. Procédé selon une des revendications 9 à 16, dans lequel un dispositif de formulation chauffant est utilisé pour la fabrication du mélange.

18. Procédé selon une des revendications 9 à 17, dans lequel une pression de plus de 0,5 * 10$^5$ Pa est exercée sur le mélange.

19. Procédé selon une des revendications 9 à 18, dans lequel la pression est exercée pendant une durée d'au moins 0,1 à 120 secondes sur le mélange.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5837275 A
- WO 0217984 A1